# EUROPÄISCHE PATENTSCHRIFT

(11) **EP 0 833 584 B1**
(45) Veröffentlichungstag und Bekanntmachung des Hinweises auf die Patenterteilung: **20.09.2000**
(21) Anmeldenummer: 96917462.2
(22) Anmeldetag: 29.05.1996
(51) Int. Cl.: A61B 5/048, G06F 19/00

(54) **VERFAHREN UND EINRICHTUNG ZUM AUSWERTEN VON ELEKTROENZEPHALOGRAMM-AUFNAHMEN**
PROCESS AND DEVICE FOR ASSESSING ELECTROENCEPHALOGRAMS
PROCEDE ET DISPOSITIF D'EXPLOITATION D'ENREGISTREMENTS D'ELECTRO-ENCEPHALOGRAMMES

(30) Priorität: 31.05.1995 DE 19519267
(43) Veröffentlichungstag der Anmeldung: 08.04.1998
(73) Patentinhaber: Herrmann, Christoph, 64291 Darmstadt (DE)
(72) Erfinder: Herrmann, Christoph, 64291 Darmstadt (DE)
(74) Vertreter: Zinngrebe, Horst, Dr.rer.nat.
(86) Internationale Anmeldenummer: EP9602309
(87) Internationale Veröffentlichungsnummer: WO9638082

(56) Entgegenhaltungen:
- EP-A- 0 137 705
- EP-A- 0 150 125
- US-A- 4 776 345

## Beschreibung

Die Erfindung betrifft ein Verfahren zum Auswerten eines elektrischen, über eine vorgegebene Zeitspanne von einer Schädeldecke abgenommenen Gehirnstromsignals sowie eine dazu geeignete Einrichtung.

Aus der deutschen Offenlegungsschrift 15 41 173 ist ein Verfahren zur Informationsverdichtung von Elektroenzephalogrammen bekannt, wobei Zeitintervalle ermittelt werden, für die der obere und untere Kurvenumkehrpunkt eines Gehirnstromsignals bestimmend ist. Aus der deutschen Offenlegungsschrift 22 47 572 ist ein Verfahren und eine Vorrichtung zum automatischen Analysieren von Gehirnstrom-Signalen bekannt, bei dem aus einem analogen EEG-Signal über einen vorgegebenen Zeitabschnitt ein Signal erzeugt wird, daß sich auf die Frequenz des EEG-Signals bezieht. Diese Methoden der Gehirnstromdarstellung haben den Nachteil, daß sie dem Arzt keine zusammenhängende, einfache und leicht überblickbare Information über die erfaßte Gehirnaktivität vermitteln.

Aus EP-A-0 150 125 ist ein Verfahren zum Auswerten elektrisch erfaßter Gehirntätigkeit bekannt, bei dem ein über eine vorgegebene Zeitspanne von einer Schädeldebke abgenommenes Signal mit einer vorgegebenen Abtastfrequenz in eine Folge von Datenwörtern umgesetzt wird, aus welchen in einem Rechenwerk nach einem vorbestimmten Schema, wie zum Beispiel einer Fourier-Transformation, die Amplituden sowie die zu jeder Amplitude gehörende Frequenz in der Weise extrahiert werden, daß Zwischendatenwörter gebildet werden, von denen jedes eine Amplitude repräsentiert und unter einer Speicheradresse abgespeichert wird. Ferner werden in der bekannten Vorrichtung aus den Zwischendatenwörtern Mittelwerte gebildet und alle Mittelwerte mit der zugehörigen Frequenz einer Anzeigeeinrichtung zur Darstellung in einem Frequenz/Amplitudendiagramm zugeleitet.

Der Erfindung liegt demgegenüber die Aufgabe zugrunde, die Beurteilung der Gehirnaktivität durch den Arzt zu erleichtern.

Dazu ist erfindungsgemäß vorgesehen, daß das Signal mit einer vorgegebenen Abtastfrequenz in eine Folge von Datenwörtern umgesetzt wird, aus welchen in einem Rechenwerk nach einem vorbestimmten Schema (Fourier-Transformation oder dergleichen) die Amplituden sowie die zu jeder Amplitude gehörende Frequenz in der Weise extrahiert werden, daß Zwischendatenwörter gebildet werden, von denen jedes eine Amplitude repräsentiert und unter einer Speicheradresse abgespeichert wird, die durch die zu dem Zwischendatenwort gehörende Frequenz bestimmt ist, wobei ferner von allen Zwischendatenwörtern diejenigen maximalen Zwischendatenwörter bestimmt werden, die ein relatives Maximum sind und daß zu jeder Frequenz aus den maximalen Zwischendatenwörtern ein Mittelwert gebildet und alle Mittelwerte mit der zugehörigen Frequenz einer Anzeigeeinrichtung zur Darstellung in einem Frequenz/Amplituden-Diagramm zugeleitet werden.

Für jede aufgenommene Spur des Elektroenzephalogramms entsteht somit ein sogenanntes Summenspektrum, aus welchem der Arzt aus der Lage der Maxima bei den einzelnen Frequenzen Rückschlüsse auf die Gehirntätigkeit ohne weiteres ziehen kann. Die Grundtätigkeit sowie eventuell pathologische Aktivität der Gehirnaktivität kann daher aus einem einzigen Kurvenverlauf entnommen und leicht medizinisch beurteilt werden.

In bevorzugter Ausgestaltung der Erfindung wird zur Mittelwertbildung die Summe der pro Frequenz vorhandenen maximalen Zwischendatenwörter mit ihrer Anzahl multipliziert und durch einen vorgegebene Bezugsgröße dividiert.

Ferner empfiehlt es sich, bei einer Unterteilung der Zeitspanne in mehrere Zeiteinheiten die Extraktion für jede Zeiteinheit (Abtastsekunde) durchzuführen, wobei die für jede Zeiteinheit gebildeten Zwischendatenwörter an Speicheradressen abgespeichert werden, die für die Zeiteinheit eineindeutig durch die zu dem Zwischendatenwort gehörende Frequenz bestimmt ist, und als Bezugsgröße die Anzahl der Zeiteinheiten in der Zeitspanne zu wählen. Bevorzugt werden die der Anzeigeeinrichtung (30) zugeführten Mittelwertdatenwörter über einen Tiefpaß geführt.

Für eine weitergehende Auswertung durch den Arzt können in Fortführung der Erfindung aus den Mittelwertdatenwörtern diejenigen ermittelt werden, die ein relatives Minimum sind, wobei die zu jedem Minimum-Mittelwertdatenwort gehörende Frequenz der Anzeigeeinrichtung zur gesonderten Darstellung zugeführt wird. Ein Frequenzdriftdiagramm läßt sich hieraus gewinnen, wenn die Frequenzen der maximalen Zwischendatenwörter in jeder Zeiteinheit ermittelt und der Anzeigeeinrichtung zugeleitet werden.

Damit ergibt sich für eine Aufnahmespur des EEGs ein einziges Diagramm, in welchem für den Arzt sofort überblickbar ist, zu welchen Zeiten während der Abtastung bei welchen Frequenzen Aktivitätsmaxima aufgetreten sind. Dies erleichtert die medizinische Auswertung gegenüber herkömmlichen Darstellungen erheblich.

Schließlich erlaubt die Erfindung eine leichte Prüfung beispielsweise des Berger-Effektes, wenn für jede Zeiteinheit alle innerhalb eines Frequenzintervalls liegenden Zwischendatenwörter der Anzeigeeinheit für eine Amplitudendriftdarstellung zugeleitet werden, wobei das Frequenz intervall durch den Frequenzabstand zweiter Minimum-Mittelwertdatenwörter definiert ist.

Zur Durchführung des beschriebenen Verfahrens eignet sich insbesondere eine Einrichtung, wie sie im Anspruch 10 angegeben ist.

Im übrigen sind bevorzugte Ausführungsformen der Erfindung in den Unteransprüchen enthalten.

Die Erfindung wird nachstehend anhand des in der beigefügten Figur dargestellten Ausführungsbeispiels im einzelnen beschrieben. Es zeigen:

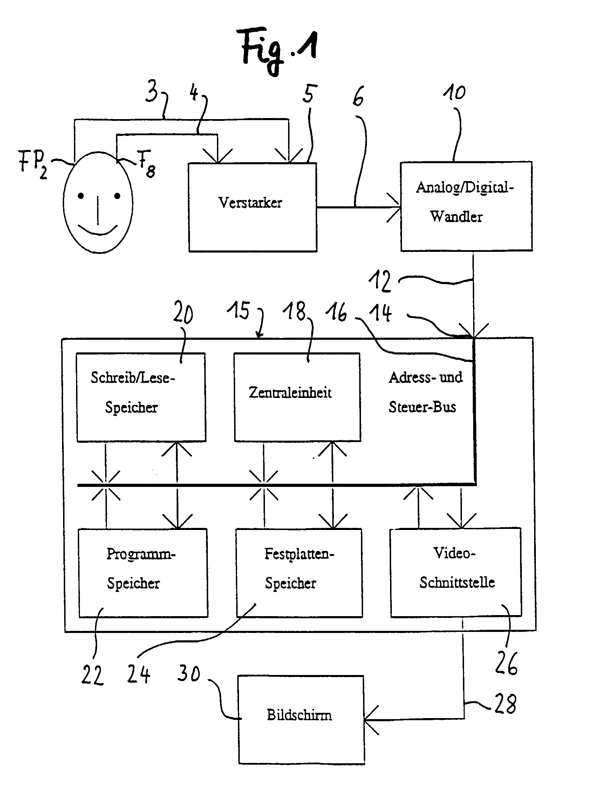

- Fig. 1: ein Blockschaltbild einer zur Durchführung des erfindungsgemäßen Verfahrens geeigneten Einrichtung;
- Fig. 2: ein über eine Zeitspanne von 10 sec. aufgenommenes Elektroenzephalogramm der Spur FP₂-F₈ ;
- Fig. 3: ein Sekundenspektrum der Sekunden null und eins aus der Aufnahme nach Fig. 2;
- Fig. 4: ein der Fig. 3 ähnliches Sekundenspektrum aus den Sekunden 2 und 3 der Aufnahme nach Fig. 2;
- Fig. 5: ein nicht gefiltertes Summenspektrum aus der Aufnahme nach Fig. 2, jedoch über eine Zeitspanne von 210 sec.
- Fig. 6: das gefilterete Summenspektrum gemäß Fig. 5;
- Fig. 7: eine Frequenzdriftdarstellung aus der Aufnahme aus Fig. 2; jedoch über die Zeitspanne von 210 sec.,
- Fig. 8: eine der Fig. 7 ähnliche Frequenzdriftdarstellung aus einer anderen Spur, und
- Fig. 9: eine Amplitendriftdarstellung.

Mit dem nachfolgend beschriebenen Ausführungsbeispiel der Erfindung werden nur diejenigen medizinischen Phänomene erfaßt, die sich im Frequenzbereich bis 16 Hz manifestieren. Bei einem Elektroenzephalogramm werden üblicherweise die Gehirnströme aus 20 Signal-Kanälen dargestellt. Die Erfindung wird zur Vereinfachung anhand der Aufnahme vornehmlich nur eines Kanals etwa in der Spur FP₂-F₈ erläutert. Selbstverständlich ist die Erfindung auf alle Spuren anwendbar, weil es für die nachstehend beschriebene Signalverarbeitung nicht auf den Entstehungsort der Signale, also die spezielle Spur ankommt. Der Arzt wird die Darstellung des Signals mit der aufgenommenen Spür in Beziehung setzen und daraus die medizinischen Schlüsse ziehen.

An der Schädeldecke eines Patienten werden an den Aufnahmestellen FP₂ und F₈ der Schädeldecke in üblicherweise Elektroden befestigt und die mit ihnen verbundenen elektrischen Leitungen 3, 4 mit den zugehörigen Eingängen eines Verstärkers 5 verbunden.

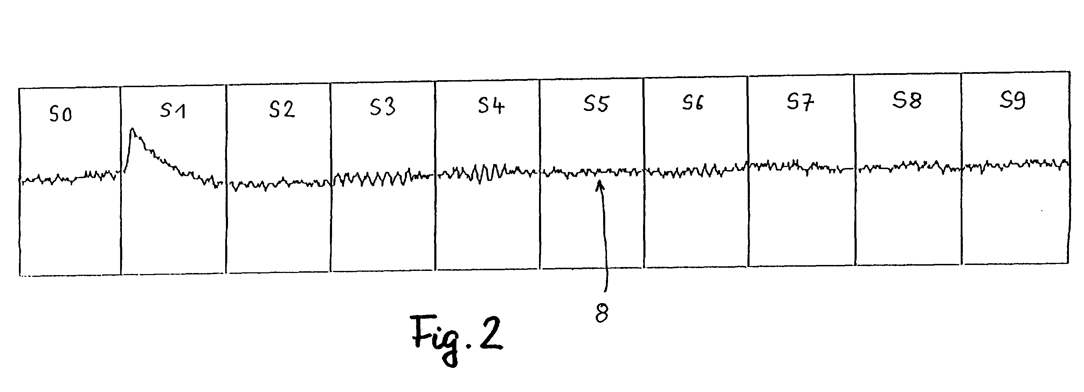

In herkömmlicher Darstellung würde der Verstärker an einer nicht dargestellten, nachgeschalteten Anzeigeeinheit einen Kurvenzug 8 zur Darstellung bringen, wie er in Fig. 2 für die ersten 10 sec. der Aufnahme wiedergegeben ist. Die gesamte Zeitspanne der Aufnahme betrage etwa 210 Sekunden, also etwa 3,5 Minuten. Die zu den ersten zehn Sekunden gehörenden Zeiteinheiten sind in Fig. 2 mit S 0...S 9 angegeben.

Das verstärkte Analogsignal 8 wird vom Verstärker 5 über eine Ausgangsleitung 6 einem Analog-Digitalwandler 10 zugeleitet, der das aufgenommene Analogsignal in 8 bit-Datenwörter umsetzt. Dazu enthält der Analog-Digital-Wandler 10 einen Abtastgenerator, der mit einer Abtastfrequenz von 128 Hz das analog ankommende Signal abtastet, wobei aus dem Amplitudenwert des Analogsignals bei jeder Abtastung das Datenwort gebildet wird. Auf der 8-adrigen Ausgangsleitung 12 erscheint daher mit einer Frequenz von 128 Hz zu jedem Abtastzeitpunkt ein Datenwort, das bitparallel dem Eingangsanschluß 14 einer Auswerteeinrichtung 15 zugeführt wird und in kodierter Form den Amplitudenwert des abgetasteten, verstärkten Signals 8 enthält.

Die Auswerteeinrichtung 15 weist eine Zentraleinheit 18, einen ersten Speicher 24, der ein Festplattenspeicher sein kann, einen Programmspeicher 22, einen zweiten Speicher 20 als Schreib/Lesespeicher und eine Videoschnittstelle 26 auf, deren Ein- und Ausgänge an einen Adress- und Steuerbus 16 angeschlossen sind. Die Videoschnittstelle 26 besitzt eine weitere Ausgangsleitung 28, welche zu einer einen Bildschirm aufweisenden Anzeigeeinrichtung 30 führt.

Unter Steuerung der Zentraleinheit 18 werden die seriell von der Ausgangsleitung 12 an den Adress- und Steuerbus 16 übergegebenen bitparallelen Datenwörter in einen ersten Speicher 24 an aufeinanderfolgenden Adressen abgespeichert. Jede Adresse des ersten Speichers repräsentiert daher denjenigen Abtastzeitpunkt, an welchem das Datenwort vom Analog/Digital-Wandler 10 generiert worden ist.

Im Programmspeicher 22 ist ein Transformationsprogramm gespeichert, welches aus einem Strom von Datenwörtern die zugehörigen Frequenzen und Amplituden extrahiert. Ein solches Programm kann beispielsweise eine Fourier-Transformation repräsentieren. Im vorliegenden Fall stellt das Programm die Maximum-Entropie-Methode dar, wie sie beispielsweise von W.H.Press et al. in "Numerical Recipes of the Art of Scientific Computing", Cambridge University Press, 1988, S. 572-576 erläutert ist.

Nachdem im ersten Speicher 24 alle über die Aufnahme-Zeitspanne von 210 Sekunden generierten 26 880 Datenwörter abgespeichert worden sind, werden diese abschnittweise unter Steuerung der Zentraleinheit 18 mit Hilfe des im Programmspeicher 22 gespeicherten Schemas transformiert und das Ergebnis der Transformation in dem zweiten Speicher 20 an den der Sekunde S 0 zugewiesenen Adressen abgespeichert. Abschnittweises speichern bedeutet hier, daß zunächst alle während der Sekunde S 0 (Fig. 2) erzeugten 128 Datenwörter transformiert und abgespeichert werden, daß im zweiten Schritt beispielsweise die während der Sekunde S 1 erzeugten Datenwörter transformiert und an der Sekunde S 1 zugewiesenen Adresse abgespeichert werden, und so weiter. Aus der Menge der Datenwörter entsteht somit eine Menge von im zweiten Speicher notierten Zwischendatenwörtern.

Im zweiten Speicher 20 sind die der Abtastsekunde S 0 zugewiesenen Adressen eineindeutig den Frequenzen f=0Hz...f=16Hz zugeordnet. Beispielsweise wird ein zur Frequenz 1Hz gehörendes Zwischendatenwort, das aus der Transformation der Datenwörter aus S 0 entstanden ist, unter der der Frequenz 1 Hz zugeordneten Adresse im zweiten Speicher abgelegt. Entsprechend werden in dem zweiten Speicher die sich aus der Transformation der Datenwörter aus der Abtastsekunde S 1 ergebenden Zwischendatenwörter unter Adressen abgespeichert, die wiederum eineindeutig den Frequenzen f=0...16Hz jetzt aber für S 1 entsprechen, und zwar jedes Zwischendatenwort unter der zu seiner Frequenz gehörenden Adresse. Jedes Zwischendatenwort enthält kodiert einen einer Amplitude proportionalen Wert. An geeigneter Stelle in einer der Einheiten 18, 20, 22 ist dazu eine Adressenliste, auf die die Zentraleinheit 18 Zugriff hat, abgespeichert, in welcher zu jeder der 210 Abtastsekunden die den Frequenzen zugeordneten Adressen enthalten sind.

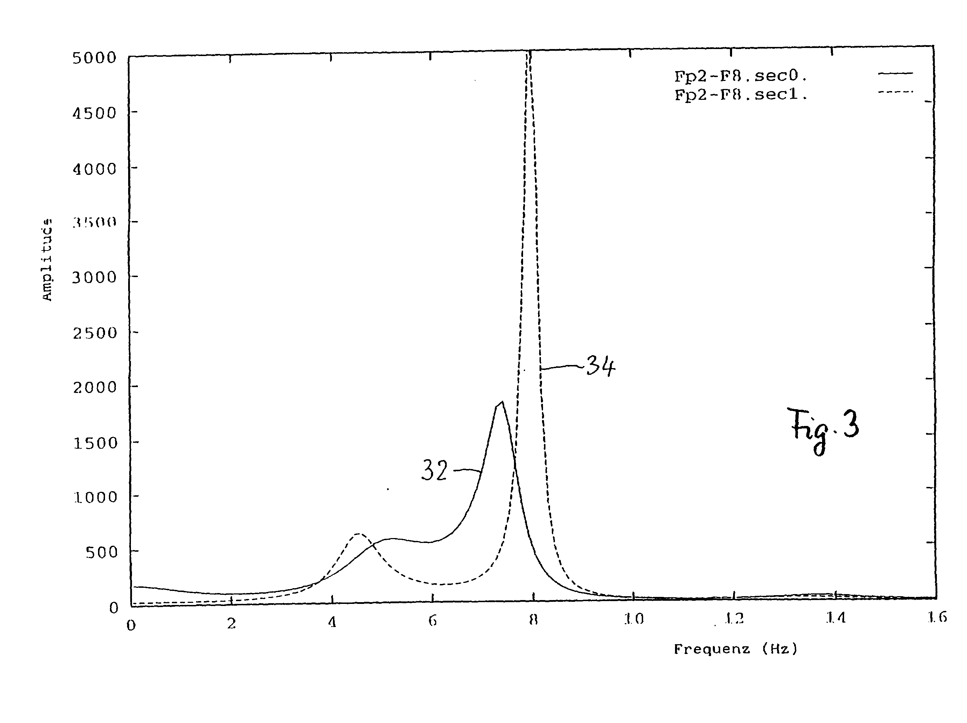

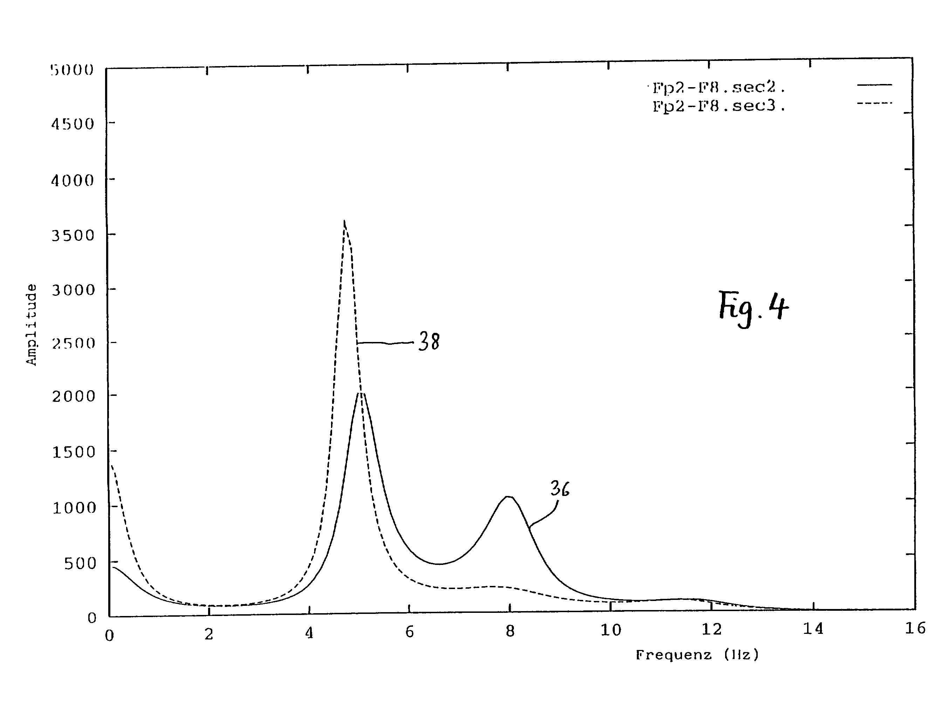

Wenn man den Inhalt des zweiten Speichers etwa in der Anzeigeeinrichtung 30 in einem Amplituden/Frequenz-Diagramm darstellen würde, würde man für die Sekunden S 0 und S 1 die in Fig. 3 dargestellten Sekundenspektren und für die Sekunden S 2 und S 3 die in Fig. 4 dargestellten Sekundenspektren erhalten. Wie in den Figuren 3 und 4 angegeben, sind die Sekundenspektren für die Sekunden S 0 und S 2 mit ausgezogenen Linien und für die Sekunden S 1 und S 3 mit gestrichelten Linien dargestellt. Man sieht, daß in S 0 die Kurve 32 ein Amplitudenmaximum bei etwa 5,2Hz und ein weiteres, höheres Amplitudenmaximum bei etwa 7,5Hz zeigt. In S 1 wurde an der Kurve 34 ein Amplitudenmaximum bei etwa 4,5Hz und ein sehr viel höheres Amplitudenmaximum bei 8Hz festgestellt. Bei S 2 der Kurve 36 findet man ein Amplitudenmaximum bei etwa 5Hz und ein weiteres bei 8Hz, und bei der Kurve 38 für S 3 schließlich ein Amplitudenmaximum bei etwa 4,7Hz und ein kleines Maximum bei etwa 8Hz. Ein drittes Maximum in den Sekundenspektren für die Sekunden 2 und 3 findet sich noch im Bereich von 11,4Hz.

Um die Auswertung des Analogsignals 8 vor allem in den Grenzbereichen zwischen den einzelnen Sekunden der Abtastzeitspanne zu verbessern, können die Abschnitte von Datenwörtern, an denen die Transformation durchgeführt wird, so gelegt werden, daß diese Abschnitte die genannten Grenzbereiche überdecken.

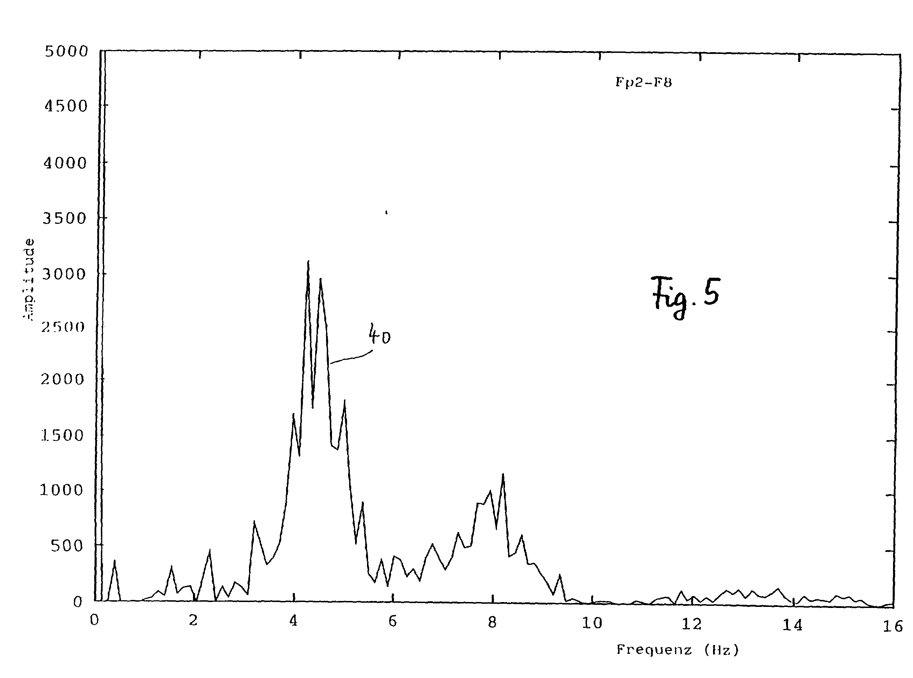

Um zu einer ersten medizinisch deutbaren Darstellung der Aufnahme gemäß dem Analogsignal 8 zu gelangen, wird aus dem Inhalt des zweiten Speichers ein Summenspektrum auf folgende Weise erzeugt: Zunächst werden für jeden Sekundenabschnitt des zweiten Speichers, also für jeden Adressenabschnitt zu S 1...S 210, durch Größenvergleich der Zwischendatenwörter diejenigen Zwischendatenwörter ermittelt, deren Wert höher ist als derjenige der beiderseits benachbarten Zwischendatenwörter. Mit anderen Worten, in jedem Sekundenabschnitt werden die Amplitudenmaxima durch die Zentraleinheit 18 ermittelt. Jedes so ermittelte maximale Zwischendatenwort wird durch die Zentraleinheit 18 markiert, beispielsweise durch Setzen eines zusätzlichen flag-bits im Zwischendatenwort. Sodann wird in einem nicht gesondert dargestellten Rechenwerk in der Zentraleinheit 18 für jede Frequenz, der eine Adresse im zweiten Speicherbereich zugordnet ist, folgende Mittelwertbildung durchgeführt: Aus allen zu einer Frequenz gehörenden markierten Zwischendatenwörtern wird die Summe aller ihrer Amplituden mit der Anzahl der zu dieser Frequenz gehörenden markierten Zwischendatenwörter multipliziert und durch die Anzahl der Abtastsekunden (210) dividiert. In einem dritten Speicherbereich des Schreib/Lese-Speichers 20 werden die so erhaltenen Mittelwertdatenwörter unter Speicheradressen, die eineindeutig der Frequenz zugeordnet sind, abgespeichert. Die Mittelwertdatenwörter sowie ihren Adressen entsprechende Signale aus dem dritten Speicherbereich des Schreib/Lese-Speichers 20 werden unter Steuerung der Zentraleinheit 18 über die Video-Schnittstelle 26 der Anzeigeeinrichtung 30 zugeleitet. Auf dem zugehörigen Bildschirm erscheint in einem Frequenz/ Amplituden-Diagramm eine in Fig. 5 wiedergegebene Kurve 40. In ihr erscheint zu jeder Frequenz der Amplitudenwert des zugehörigen Mittelwertdatenwortes.

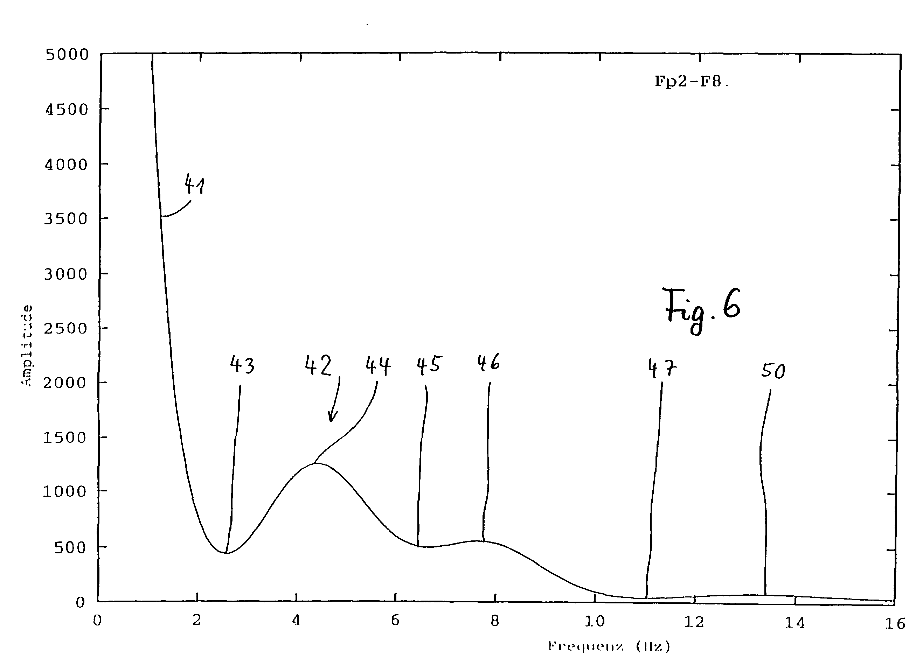

Wie aus Fig. 5 zu erkennen ist, zeichnet sich die Kurve 40 durch eine Vielzahl teils sehr spitzer Zacken aus. Um hier dem Arzt die Auswertung zu erleichtern, werden die Mitteldatenwörter aus dem dritten Speicherbereich über ein Tiefpaßfilter geführt und erst dann der Videoschnittstelle 26 über den Adress- und Steuerbus 16 zugeleitet, wodurch die Kurve 40 zu der in Fig. 6 dargestellten Summenkurve 42 geglättet wird. Abgesehen von einem Gleichstromanteil am Ast 41 der Kurve 42 im Frequenzbereich unter 1,5Hz zeigt sich dem Betrachter ein erstes relatives Maximum 44 bei etwa 4Hz und ein kleineres zweites Maximum 46 bei etwa 8Hz. Ein kaum wahrnehmbares drittes Maximum 50 ist bei etwa 15Hz zu notieren.

Medizinisch bedeutet dieses Ergebnis, daß bei dem untersuchten Patienten eine wesentliche Gehirnaktivität bei 4Hz und eine geringere bei 8Hz vorliegt. Vor allem das erste Maximum 44 liegt deutlich unterhalb des Frequenzbereichs von 8-13Hz der Alpha-Wellen, was auf eine pathologische Situation in dem Gehirnabschnitt des Patienten hindeutet, der der aufgenommenen Position entspricht.

Um die Aussagekraft der Darstellung des Analogsignals 8 für den Mediziner noch weiter zu erhöhen, wird unter Zuhilfenahme der im ersten Speicher 24 gespeicherten Datenwörter eine Frequenzdriftdarstellung des Analogsignals erstellt, die es erlaubt, die extrahierten Frequenzen mit der medizinischen Einteilung in die bereits erwähnten Alpha-Wellen (Frequenzbereich von 8 bis 13Hz) die Beta-Wellen (Frequenz über 13Hz) sowie dem Delta-Band (Frequenzen unter 3Hz) und dem Theta-Band (Frequenzen von 4-7Hz) zu vergleichen.

Wie man nämlich aus Fig. 6 erkennt, findet verstärkte Gehirnaktivität, repräsentiert durch die Maxima 44, 46 und 50 innerhalb von Frequenzbändern statt, deren Grenzen durch die zwischen den Maxima liegenden Minima gegeben sind. So findet sich ein relatives Minimum 43 bei etwa 2,5 Hz, ein weiteres Minimum 45 bei etwa 6,2 Hz und ein drittes Minimum 47 bei etwa llHz. Daher wird in der Zentraleinheit 18 aus dem dritten Speicherbereich im Schreib/Lese-Speicher 20 etwa mittels des erwähnten Vergleichers festgestellt, bei welchen Frequenzen relative Minima der Mittelwertdatenwörter anzutreffen sind.

Die zugehörigen Frequenzen Fₘᵢₙ bzw. die diesen entsprechende Signale werden über die Videoschnittstelle 26 der Anzeigeeinrichtung 30 zugeleitet und auf dem Bildschirm in einem Diagramm, auf dessen Abszisse die gesamte Aufnahme-Zeitspanne in Sekunden und auf dessen Ordinate die Frequenz in Hz abgetragen ist, als horizontale Linien 53, 55, 57 dargestellt.

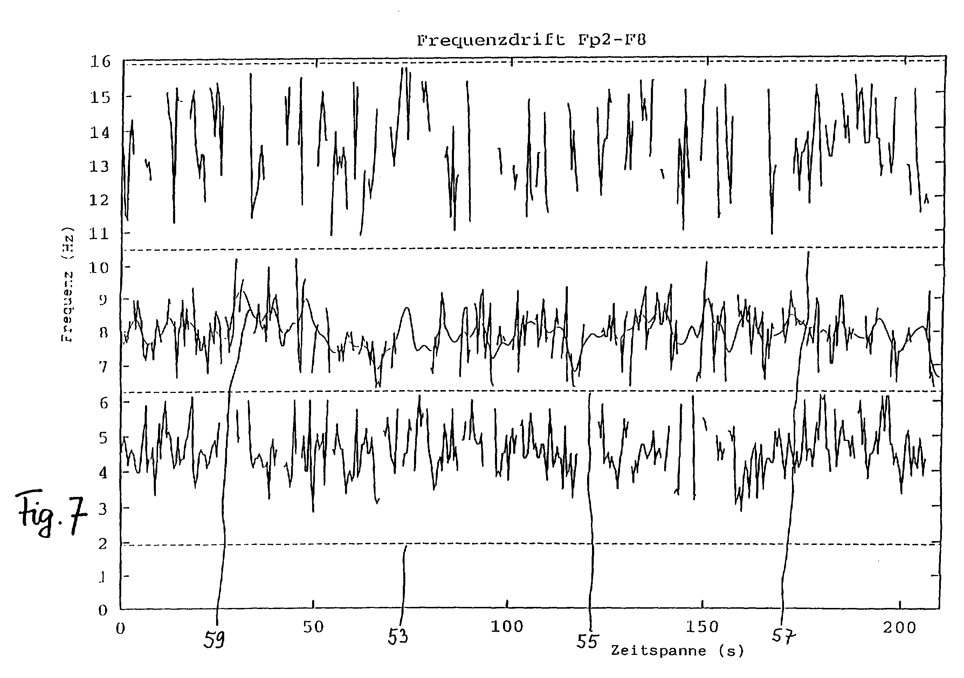

Ferner wird von der Zentraleinheit 18 auf den zweiten Speicher 20 zugegriffen und innerhalb jedes Sekundenabschnittes alle Zwischendatenwörter auf das Vorhandensein der Markierung (die, wie erwähnt, ein relatives Maximum anzeigt) geprüft. Die zu den markierten Zwischendatenwörtern gehörenden Adressen (die eine Frequenz repräsentieren) werden als zu dem jeweiligen Sekundenabschnitt gehörende (Frequenz-)Punkte in das Diagramm der Figur 7 eingetragen, indem die jeweiligen Signale der Anzeigeeinrichtung 30 zugeführt werden. Die Anzeigeeinheit 30 erhält also für jeden Sekundenabschnitt S 0, S 1,...S 210 im zweiten Speicherbereich ein oder mehrere Frequenzsignale, von denen jedes einem ein Amplitudenmaximum repräsentierenden Zwischendatenwort zugeordnet ist. Diese Frequenzsignale trägt die Anzeigeeinrichtung 30 in das in Fig. 7 dargestellte Diagramm ein. Man erkennt innerhalb der einzelnen Frequenzbereiche, angezeigt durch die gestrichelten Linien 53, 55, 57 über die Aufnahmezeitspanne von 210 Sekunden mehrere unterbrochene Kurvenzüge.

Um in dem Diagramm der Fig. 7 insbesondere im Bereich des Grundrhythmus eine bessere Übersicht zu gewinnen, werden die in diesen Frequenzbereich fallenden Frequenzsignale gesondert über einen Tiefpass geführt und das Ausgangssignal des Tiefpasses in dem Frequenzbereich zwischen den Kurven 55 und 57 durch die Anzeigeeinrichtung 30 eingetragen. Man erhält die dargestellte Integrationskurve 59. Der Grundrhytmus ist durch dasjenige Frequenzintervall zwischen den Kurven 53, 55, 57 definiert, in welchem die Anzahl der auftretenden Frequenzsignale maximal ist.

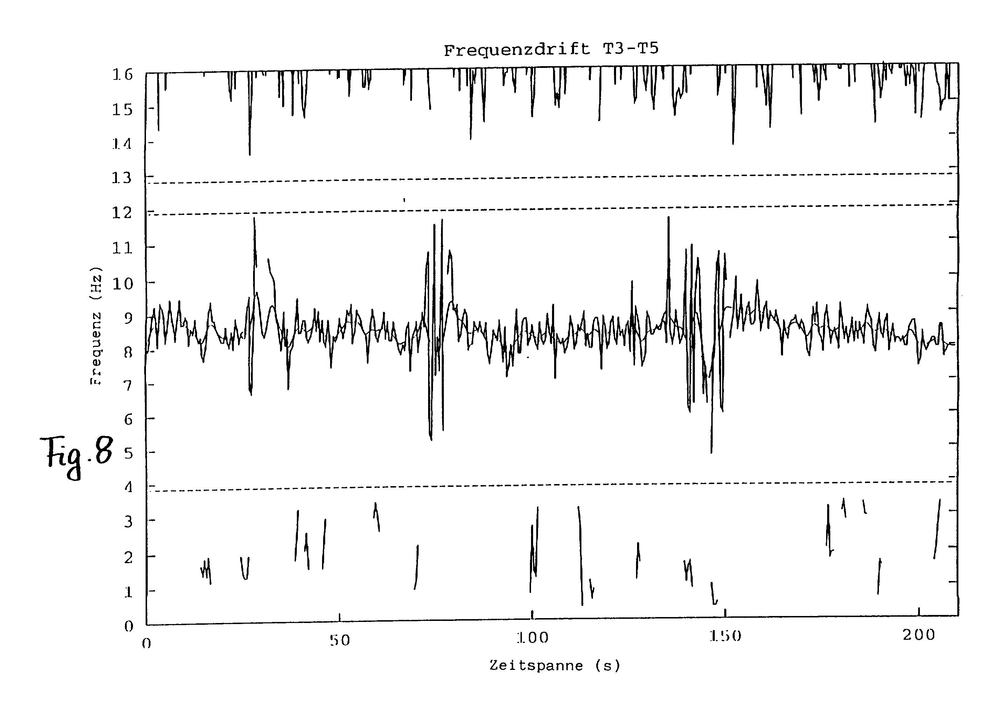

Aus dem Frequenzdriftdiagramm für die Spur FP₂-F₈ der Fig. 7 erkennt der Arzt, daß der dem Alpha-Wellenbereich zugeordnete Grundrhythmus an dem erfaßten Teil der Hemisphäre des Patienten generalisiert leicht verlangsamt ist, weil der Grundrhythmus der Gehirnaktivität hier im Frequenzbereich von etwa 6,5 bis etwa 10,5 Hz und die Integrationskurve sehr oft unter 8 Hz liegt, während der Grundrhythmus beim Gesunden im Alpha-Bereich von 8 bis 13 Hz anzutreffen ist. Ferner ist für den Arzt aus diesem Frequenzdriftdiagramm abzulesen, daß bei dem untersuchten Patienten ein kontinuierlich mittelschwerer epileptogener Herdbefund rechts, temporal, vorliegt. In einer anderen Spur T3-T5 (Fig. 8) kann man erkennen, daß keine Aktivität im Bereich von 4 bis 7 Hz vorhanden ist. Der Grundrhythmus ist auch leicht verlangsamt und die Aktivität im Bereich unter 4Hz ist Bulbusartefakten zuzuschreiben. Durch den Vergleich der Frequenzdriftdiagramme verschiedener Hirnregionen kann der Arzt zwischen einem Herdbefund und einem generalisierten Phänomen unterschieden.

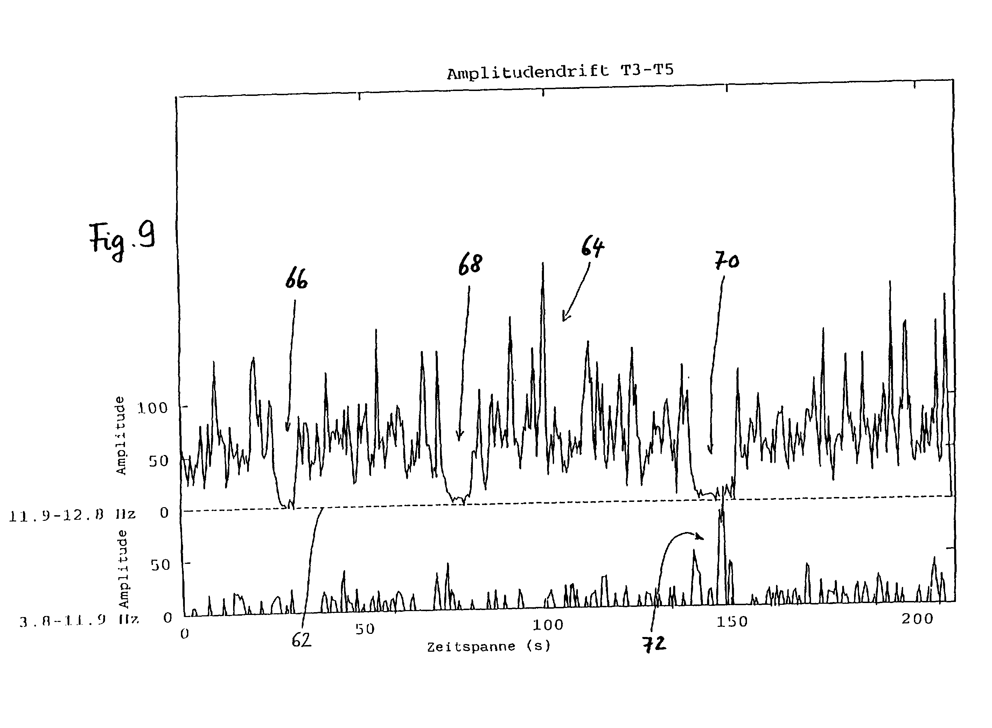

Schließlich läßt sich der Inhalt des zweiten Speichers noch als Amplitudendrift darstellen, der dem Arzt weitere Aufschlüsse über die Gehirntätigkeit vermittelt. Dazu greift die Zentraleinheit 18 wieder auf den zweiten Speicher 20 zu und ermittelt sekundenweise, also zunächst für S 0, sodann für S 1 und so weiter, diejenigen Zwischendatenwörter, die innerhalb eines vorgegebenen Frequenzbereichs also eines vorgegebenen Adressenunterabschnittes liegen. Jeder Adressenunterabschnitt ist definiert durch das Frequenzintervall zwischen zwei relativen Minima im Summenspektrum der Fig. 6, also durch die Frequenzgrenzen 53, 55, 57 aus Fig. 7. Bei einer EEG-Aufnahme an der Spur T3-T5 (Fig. 8) liegen die Frequenzintervalle bei 3,8...11,9Hz sowie bei 11,9...12,8Hz. Zu jedem der in jedem Frequenzintervall vorgefundenen Zwischendatenwörter mit Markierung (Maximum) bildet die Zentraleinheit 18 ein seinem Wert proportionales Signal und sendet dieses an die Anzeigeeinrichtung 30. Die Anzeigeeinrichtung 30 trägt diese Signale in ein Diagramm gemäß Fig. 9 ein, auf dessen Abszisse wieder die gesamte Aufnahme-Zeitspanne von 210 Sekunden und auf dessen Ordinate die Amplituden in uVolt, für jeden Frequenzbereich bezogen auf eine eigene Nullinie, abgetragen sind. Über der Referenzlinie 62 zum Frequenzbereich 11,9...12,8Hz erscheint dann über die gesammte Aufnahme-Zeitspanne (S1,...,S20) der Kurvenzug 64. In diesem Kurvenzug 64 sind bei 66, 68 und 70 sogenannte dropouts festzustellen, die sich durch Amplitudenwerte nahe null uVolt kennzeichnen. Durch Vergleich der zeitlichen Lage der Dropouts 66, 68 und 70 mit dem Aufnahmeprotokoll wird sich ergeben, daß während der zugehörigen Aufnahmezeiten der Patient die Augen geöffnet hatte. Der dreimalige Berger-Effekt bei den Aufnahmezeiten von 30, 70 und 140 Sekunden ist medizinisch also positiv zu bewerten.

Schließlich lassen sich an dem Amplitudendriftdiagramm der Fig. 9 bei den Aufnahmezeiten von 140-150sec. im Frequenzbereich von 3,8-11,9Hz hochamplitudige Bulbusartefakte 72 feststellen.

Man sieht, daß sich durch die vorstehend beschriebene Art der Verarbeitung von EEG-Signalen mit der Auswerteeinrichtung 15 und dem Analog-Digital-Wandler 10 Darstellungen der Gehirnaktivität in der Anzeigeeinrichtung 30 gewinnen lassen, die dem Arzt leicht und ohne weiteres eine medizinische Beurteilung ermöglichen.

Frequenzgebundene Phänomene, wie der Grundrhythmus, pathologisch langsame Aktivität oder bestimmte Artefakte, werden in getrennten Frequenzbändern einzeln beschrieben und sind für den Arzt individuell auswertbar und stören sich nicht gegenseitig wie im herkömmlichen EEG.

## Patentansprüche

1. Verfahren zum Auswerten elektrisch erfaßter Gehirnaktivität, bei dem ein über eine vorgegebene Zeitspanne von einer Schädeldecke abgenommenes Signal mit einer vorgegebenen Abtastfrequenz in eine Folge von Datenwörtern umgesetzt wird, aus welchen in einem Rechenwerk nach einem vorbestimmten Schema, wie beispielsweise einer Fournier-Transformation, die Amplituden sowie die zu jeder Amplitude gehörende Frequenz in der Weise extrahiert werden, daß Zwischendatenwörter gebildet werden, von denen jedes eine Amplitude repräsentiert und unter einer Speicheradresse abgespeichert wird, die durch die zu dem Zwischendatenwort gehörende Frequenz bestimmt ist, wobei ferner von allen Zwischendatenwörtern diejenigen maximalen Zwischendatenwörter bestimmt werden, die ein relatives Maximum sind und daß zu jeder Frequenz aus den maximalen Zwischendatenwörtern ein Mittelwert gebildet und alle Mittelwerte mit der zugehörigen Frequenz einer Anzeigeeinrichtung (30) zur Darstellung in einem Frequenz/Amplitudendiagramm zugeleitet werden.

2. Verfahren nach Anspruch 1, dadurch gekennzeichnet, daß zur Mittelwertbildung die Summe der pro Frequenz vorhandenen maximalen Zwischendatenwörter mit ihrer Anzahl multipliziert und durch eine vorgegebene Bezugsgröße dividiert wird.

3. Verfahren nach Anspruch 1 oder 2, dadurch gekennzeichnet, daß jeder Mittelwert als Mittelwertdatenwort an einer durch die zugehörige Frequenz bestimmten Adresse vorübergehend gespeichert wird.

4. Verfahren nach einem oder mehreren der vorstehenden Ansprüche, dadurch gekennzeichnet, daß bei einer Unterteilung der Zeitspanne in mehrere Zeiteinheiten die Extraktion für jede Zeiteinheit bzw. Abtastsekunde durchgeführt wird, wobei die für jede Zeiteinheit gebildeten Zwischendatenwörter an Speicheradressen abgespeichert werden, die für die Zeiteinheit eineindeutig durch die zu dem Zwischendatenwort gehörende Frequenz bestimmt ist, und daß die Bezugsgröße die Anzahl der Zeiteinheiten in der Zeitspanne ist.

5. Verfahren nach einem oder mehreren der vorstehenden Ansprüche, dadurch gekennzeichnet, daß die der Anzeigeeinrichtung (30) zugeführten Mittelwertdatenwörter über einen Tiefpaß geführt werden.

6. Verfahren nach einem oder mehreren der vorstehenden Ansprüche, dadurch gekennzeichnet, daß aus den Mittelwertdatenwörtern diejenigen ermittelt werden, die ein relatives Minimum sind, und daß die zu jedem Minimum-Mittelwertdatenwort gehörende Frequenz der Anzeigeeinrichtung (30) zur gesonderten Darstellung zugeführt wird.

7. Verfahren nach einem oder mehreren der vorstehenden Ansprüche, dadurch gekennzeichnet, daß die Frequenzen der maximalen Zwischendatenwörter in jeder Zeiteinheit ermittelt und der Anzeigeeinrichtung (30) zu einer Frequenzdriftdarstellung zugeleitet werden.

8. Verfahren nach Anspruch 7, dadurch gekennzeichnet, daß die Frequenzen, die im Bereich des Grundrhythmus liegen, über einen Tiefpaß geführt werden und der Ausgang des Tiefpasses der Anzeigeeinheit (30) zugeleitet wird.

9. Verfahren nach einem oder mehreren der vorstehenden Ansprüche, dadurch gekennzeichnet, daß für jede Zeiteinheit alle innerhalb eines Frequnezintervalls liegenden Zwischendatenwörter der Anzeigeeinheit (30) für eine Amplitudendriftdarstellung zugeleitet werden, wobei das Frequenzintervall durch den Frequenzabstand zweier Minimum-Mittelwertdatenwörter definiert ist.

10. Einrichtung zum Auswerten elektrisch erfaßter Gehirnaktivität zur Durchführung des Verfahrens nach einem der vorstehenden Ansprüche, mit einem Analog/ Digital-Wandler (10), in welchem ein über eine vorgegebene Zeitspanne von einer Schädeldecke abgenommenes Signal (8) mit einer vorgegebenen Abtastfrequenz in eine Folge von Datenwörtern umgesetzt wird, mit einer Auswerteeinrichtung (15), deren Adreß- und Steuerbus (16) die Datenwörter aus dem Analog/Digital-Wandler (10) einem ersten Speicher (24) zuführt, wobei in einem Programmspeicher (22) der Auswerteeinrichtung (15) ein vorbestimmtes Schema, wie beispielsweise einer Fournier-Transformation, gespeichert ist, mit welchem eine in der Auswerteeinrichtung (15) vorgesehene und ein Rechenwerk enthaltende Zentraleinheit (18) aus den im ersten Speicher (24) gespeicherten Datenwörtern die Amplituden sowie die zu jeder Amplitude gehörende Frequenz extrahiert und in einem zweiten Speicher (20) in der Weise abspeichert, daß unter Adressen, die den Frequenzen zugeordnet sind, Zwischendatenwörter eingeschrieben werden, von denen jedes eine Amplitude repräsentiert, wobei das Rechenwerk diejenigen Zwischendatenwörter ermittelt, die ein relatives Maximum sind und zu jeder Frequenz aus den maximalen Zwischendatenwörtern einen Mittelwert bildet, und wobei die Zentraleinheit (18) alle Mittelwerte mit den zugehörigen Frequenzen über den Adressen- und Steuerbus (16) einer Anzeigeeinrichtung (30) zuführt.

11. Einrichtung nach Anspruch 10, dadurch gekennzeichnet, daß die Anzeigeeinrichtung (30) einen Bildschirm aufweist und über eine Videoschnittstelle (26) mit dem Adressen- und Steuerbus (26) gekoppelt ist.

12. Einrichtung nach Anspruch 10 oder 11, dadurch gekennzeichnet, daß das Rechenwerk zur Mittelwertbildung die Summe der pro Frequenz vorhandenen maximalen Zwischendatenwörter mit ihrer Anzahl multipliziert und durch eine vorgegebene Bezugsgröße dividiert.

13. Einrichtung nach einem der Ansprüche 10 bis 12, dadurch gekennzeichnet, daß die Zentraleinheit (18) jeden Mittelwert als Mittelwertdatenwort an einer durch die zugehörige Frequenz bestimmten Adresse eines dritten Speichers vorübergehend abspeichert.

14. Einrichtung nach einem oder mehreren der Ansprüche 10 bis 13, dadurch gekennzeichnet, daß die Auswerteeinrichtung (15) einen Tiefpaß aufweist, über welchen die Zentraleinheit (18) die Mittelwertdatenwörter der Anzeigeeinrichtung (30) zuführt.

15. Einrichtung nach einem oder mehreren der Ansprüche 10 bis 14, dadurch gekennzeichnet, daß das Rechenwerk aus den Mittelwertdatenwörter diejenigen ermittelt, die ein relatives Minimum sind, und daß die Zentraleinheit (18) die zu jedem Minimum-Mittelwertdatenwort gehörende Frequenz der Anzeigeeinrichtung (30) zuführt.

16. Einrichtung nach einem oder mehreren der Ansprüche 10 bis 15, dadurch gekennzeichnet, daß bei einer Einteilung der Zeitspanne in mehrere Zeiteinheiten das Rechenwerk die Extraktion für jede Zeiteinheit bzw. Abtastsekunde durchführt und die Zentraleinheit (18) die für jede Zeiteinheit gebildeten Zwischendatenwörter an Speicheradressen des zweiten Speichers (20) abspeichert, die für die Zeiteinheit eineindeutig durch die zu dem Zwischendatenwort gehörende Frequenz bestimmt ist, und daß die Bezugsgröße die Anzahl der Zeiteinheiten ist.

17. Einrichtung nach einem oder mehreren der Ansprüche 10 bis 16, dadurch gekennzeichnet, daß das Rechenwerk für jede Zeiteinheit alle innerhalb eines Frequenzintervalls liegenden Zwischendatenwörter der Anzeigeeinrichtung (30) zuleitet, wobei das Frequenzintervall durch den Frequenzabstand zweier Minimum-Mittelwertdatenwörter bestimmt ist.

18. Einrichtung nach einem oder mehreren der Ansprüche 10 bis 17, dadurch gekennzeichnet, daß die Zentraleinheit (18) eine Liste von Speicheradressen mit zugehörigen Frequenzen aufweist.

19. Einrichtung nach einem oder mehreren der Ansprüche 10 bis 18, dadurch gekennzeichnet, daß das Rechenwerk für jede Zeiteinheit alle innerhalb eines dem Grundrhythmus zugeordneten Frequenzintervalls liegenden Frequenzen der maximalen Zwischendatenwörter über einen Tiefpaß der Anzeigeeinrichtung (30) zuleitet.

## Claims

1. Method for evaluating electrically detected brain activity, in which a signal picked off from the top of the skull over a given time interval is converted at a given sampling frequency into a sequence of data words from which the amplitudes and the frequency associated with each amplitude are extracted in accordance with a predetermined scheme like for instance the Fourier transformation in an arithmetic unit, in such a manner that intermediate data words are formed, each of which represents an amplitude and is saved at a memory address which is governed by the frequency associated with the intermediate data word, furthermore, of all the intermediate data words, those maximum intermediate data words being determined which are a relative maximum, and in such a manner that a mean value is formed from the maximum intermediate data words for each frequency and all the mean values are passed with the associated frequency to an indicating device (30) for display using a frequency/amplitude graph.

2. Method according to claim 1, characterized in that, in order to form the mean value, the sum of the maximum intermediate data words present per frequency is multiplied by the number of said words and is divided by a given reference variable.

3. Method according to claim 1 or 2, characterized in that each mean value is temporarily saved, as a mean value data word, at an address governed by the associated frequency.

4. Method according to one or more of the preceding claims, characterized in that with the time interval being subdivided into a number of time units, the extraction is carried out for each time unit and respectively sampling second, the intermediate data words formed for each time unit being saved at memory addresses which are defined unambiguously for the time unit by the frequency associated with the intermediate data word, and in that the reference variable is the number of time units in the time interval.

5. Method according to one or more of the preceding claims, characterized in that the mean value data words fed to the indicating device (30) are passed via a low-pass filter.

6. Method according to one or more of the preceding claims, characterized in that, of the mean value data words, those are determined which are a relative minimum, and in that the frequency associated with each minimum mean value data word is fed to the indicating device (30) for separate display.

7. Method according to one or more of the preceding claims, characterized in that the frequencies of the maximum intermediate data words in each time unit are determined and are passed to the indicating device (30) to provide a frequency drift display.

8. Method according to claim 7, characterized in that the frequencies which are in the region of the basic rhythm are passed via a low-pass filter, and the output of the low-pass filter is fed to the indicator unit (30).

9. Method according to one or more of the preceding claims, characterized in that, for each time unit, all the intermediate data words within a frequency interval are passed to the indicator unit (30) to provide an amplitude drift display, the frequency interval being defined by the frequency difference between two maximum mean value data words.

10. Device for evaluating electrically detected brain activity for carrying out the method according to one of the preceding claims, having an analog/digital converter (10) in which a signal (8) picked off from the top of the skull over a given time interval is converted at a given sampling frequency into a sequence of data words, having an evaluation device (15) whose address and control bus (16) feeds the data words from the analog/digital converter (10) to a first memory (24), a predetermined scheme like for instance the Fourier transformation being stored in a program memory (22) in the evaluation device (15), by means of which scheme a central processor (18) which is provided in the evaluation device (15), and contains an arithmetic unit, extracts from the data words stored in the first memory (24) the amplitudes as well as the frequency associated with each amplitude and saves them in a second memory (20) in such a manner that the addresses which are assigned to the frequencies have intermediate data words written to them, each of which represents an amplitude, the arithmetic unit determining those intermediate data words which are relative maximum and forming a mean value for each frequency from the maximum intermediate data words, and the central processor (18) feeding all the mean values, with the associated frequencies, via the address and control bus (16) to an indicating device (30).

11. Device according to claim 10, characterized in that the indicating device (30) has a screen and is coupled to the address and control bus (26) via a video interface (26).

12. Device according to claim 10 or 11, characterized in that, in order to form the mean value, the arithmetic unit multiplies the sum of the maximum intermediate data words present per frequency by the number of said data words, and divides it by a given reference variable.

13. Device according to one claims 10 to 12, characterized in that the central processor (18) temporarily saves each mean value as a mean value data word at an address, governed by the associated frequency, in a third memory.

14. Device according to one or more of claims 10 to 13, characterized in that the evaluation device (15) has a low-pass filter via which the central processor (18) feeds the mean value data words to the indicating device (30).

15. Device according to one or more of claims 10 to 14, characterized in that the arithmetic unit determines from the mean value data words those which are a relative minimum, and in that the central processor (18) feeds to the indicating device (30) the frequency associated with each minimum mean value data word.

16. Device according to one or more of claims 10 to 15, characterized in that, with the time interval being split into a number of time units, the arithmetic unit carries out the extraction for each time unit and respectively sampling second and the central processor (18) saves the intermediate data words formed for each time unit at memory addresses in the second memory (20), which is are unambiguously defined for the time unit by the frequency associated with the intermediate data word, and in that the reference variable is the number of time units.

17. Device according to one or more of claims 10 to 16, characterized in that the arithmetic unit passes, for each time unit, all the intermediate data words within a frequency interval to the indicating device (30), the frequency interval being foverned by the frequency difference between two minimum mean value data words.

18. Device according to one or more of claims 10 to 17, characterized in that the central processor (18) has a list of memory addresses with associated frequencies.

19. Device according to one or more of claims 10 to 18, characterized in that the arithmetic unit passes to the indicating device (30), for each time unit and via a low-pass filter, all those frequencies of the maximum intermediate data words which are within a frequency interval associated with the basic rhythm.

## Revendications

1. Méthode pour évaluer l'activité du cerveau détectée électriquement, d'après laquelle un signal prélevé sur la boîte crânienne pendant une période de temps donnée est converti à une fréquence d'échantillonnage prédéterminée en une séquence de mots porteurs d'information ou mots-données d'où sont extraits les amplitudes et aussi la fréquence correspondant à chaque amplitude dans une unité de calcul suivant un schéma prédéterminé, comme par exemple la transformation de Fourier, de telle façon que des mots-données intermédiaires sont formés, chacun d'entre eux représentant une amplitude et étant mémorisé à une adresse de mémoire qui est déterminée par la fréquence correspondant au mot-donnée intermédiaire, en outre de tous les mots-données intermédiaires, les mots-données intermédaires maximum déterminés sont ceux qui sont un maximum relatif et de telle façon que pour chaque fréquence une valeur moyenne est formée à partir des mots-données intermédiaires maximum et toutes les valeurs moyennes sont amenées à un dispositif d'affichage (30) à la fréquence correspondante pour être représentées sur un graphique amplitude/fréquence.

2. Méthode selon la revendication 1, caractérisée en ce que pour former une valeur moyenne la somme des mots-données intermédiaires maximum en présence par fréquence est multipliée par leur nombre et divisée par une grandeur de référence donnée.

3. Méthode selon la revendication 1 ou 2, caractérisée en ce que chaque valeur moyenne est sauvegardée provisoirement en tant que mot-donnée de valeur moyenne à une adresse déterminée par la fréquence correspondante.

4. Méthode selon une ou plusieurs des revendications précédentes, caractérisée en ce qu'en subdivisant la période de temps en plusieurs unités de temps l'extraction a lieu pour chaque unité de temps, et respectivement pour chaque seconde d'échantillonnage, les mots-données intermédiaires formés pour chaque unité de temps étant mémorisés à des adresses de mémoire qui sont clairement déterminées pour l'unité de mémoire par la fréquence correspondant au mot-donnée intermédiaire et en ce que la grandeur de référence est le nombre des unités de temps dans la période de temps.

5. Méthode selon une ou plusieurs des revendications précédentes, caractérisée en ce que les mots-données de valeur moyenne amenés au dispositif d'affichage (30) sont passés par un filtre passe-bas.

6. Méthode selon une ou plusieurs des revendications précédentes, caractérisée en ce que parmi les mots-données de valeur moyenne ceux qui sont détectés sont un minimum relatif et que la fréquence correspondant à chaque mot-donnée de valeur moyenne minimum est amené à un dispositif d'affichage (30) pour une représentation séparée.

7. Méthode selon une ou plusieurs des revendications précédentes, caractérisée en ce que les fréquences des mots-données intermédiaires maximum sont détectées dans chaque unité de temps et amenées au dispositif d'affichage (30) pour une représentation de déviation de fréquence.

8. Méthode selon la revendication 7, caractérisée en ce que les fréquences qui sont dans le domaine du rythme de base, sont passés par un filtre passe-bas et que la sortie du filtre passe-bas est amenée à l'unité d'affichage (30).

9. Méthode selon une ou plusieurs des revendications précédentes, caractérisée en ce que pour chaque unité de temps tous les mots-données intermédiaires placés dans un intervalle de fréquence sont amenés à l'unité d'affichage (30) pour une représentation de déviation d'amplitude, l'intervalle de fréquence étant défini par l'écart de fréquence entre deux mots-données de valeur moyenne minimum.

10. Dispositif pour évaluer l'activité du cerveau détectée électriquement en mettant en oeuvre la méthode selon une des revendications précédentes, avec un convertisseur analogique-numérique (10) dans lequel un signal (8) prélevé sur une boîte crânienne pendant une période de temps donnée à une fréquence d'échantillonnage prédéterminée est converti en une séquence de mots-données, avec un dispositif d'évaluation (15) dont le bus d'adresses et de commande (16) apporte les mots-données du convertisseur analogique-numérique (10) à une première mémoire (24), un schéma prédéterminé, comme par exemple la transformation de Fourier, étant mémorisé dans une mémoire de programme (22) du dispositif d'évaluation (15), avec lequel un processeur central (18), prévu dans le dispositif d'évaluation (15) et comprenant une unité de calcul, extrait les amplitudes des mots-données mémorisés dans la première mémoire (24) de même que la fréquence correspondant à chaque amplitude et les mémorise dans une deuxième mémoire (20) de telle manière qu'à des adresses qui sont attribuées aux fréquences, des mots-données intermédiaires sont inscrits dont chacun représente une amplitude, l'unité de calcul détectant les mots-données intermédiaires qui sont un maximum relatif et formant une valeur moyenne pour chaque fréquence à partir des mots-données intermédiaires maximum et le processeur central (18) apportant toutes les valeurs moyennes avec les fréquences correspondantes à un dispositif d'affichage (30) en passant par l'intermédiaire du bus d'adresses et de commande (16).

11. Dispositif selon la revendication 10, caractérisé en ce que le dispositif d'affichage (30) présente un écran et est accouplé au bus d'adresses et de commande (26) par l'intermédiaire d'une interface vidéo (26).

12. Dispositif selon la revendication 10 ou 11, caractérisé en ce que l'unité de calcul pour former la valeur moyenne multiplie la somme des mots-données intermédiaires maximum existant par fréquence par leur nombre et la divise par une grandeur de référence donnée.

13. Dispositif selon une des revendications 10 à 12, caractérisé en ce que le processeur central (18) sauvegarde provisoirement chaque valeur moyenne en tant que mot-donnée de valeur moyenne à une adresse d'une troisième mémoire déterminée par la fréquence correspondante.

14. Dispositif selon une ou plusieurs des revendications 10 à 13, caractérisé en ce que le dispositif d'évaluation (15) présente un filtre passe-bas par l'intermédiaire duquel le processeur central (18) apporte les mots-données de valeur moyenne à un dispositif d'affichage (30).

15. Dispositif selon une ou plusieurs des revendications 10 à 14, caractérisé en ce que parmi les mots-données de valeur moyenne ceux que l'unité de calcul détecte sont un minimum relatif et en ce que le processeur central (18) amène la fréquence correspondant à chaque mot-donnée de valeur moyenne minimum au dispositif d'affichage (30).

16. Dispositif selon une ou plusieurs des revendications 10 à 15, caractérisé en ce que pour une division de la période de temps en plusieurs unités de temps, l'unité de calcul effectue l'extraction pour chaque unité de temps et respectivement chaque seconde d'échantillonnage et que le processeur central (18) sauvegarde les mots-données intermédiaires formés pour chaque unité de temps à des adresses de mémoires de la deuxième mémoire (20) qui est déterminée clairement pour l'unité de temps par la fréquence correspondant au mot-donnée intermédiaire et que la grandeur de référence est le nombre d'unités de temps.

17. Dispositif selon une ou plusieurs des revendications 10 à 16, caractérisé en ce que, pour chaque unité de temps, l'unité de calcul amène tous les mots-données placés dans un intervalle de fréquence à un dispositif d'affichage (30), l'intervalle de fréquence étant déterminé par l'écart de fréquence entre deux mots-données de valeur moyenne minimum.

18. Dispositif selon une ou plusieurs des revendications 10 à 17, caractérisé en ce que le processeur central (18) présente une liste d'adresses de mémoire avec fréquences correspondantes.

19. Dispositif selon une ou plusieurs des revendications 10 à 18, caractérisé en ce que l'unité de calcul amène pour chaque unité de temps toutes les fréquences des mots-données intermédiaires maximum placées dans un intervalle de fréquence attribué au rythme de base par l'intermédiaire d'un filtre passe-bas du dispositif d'affichage (30).
